(19) Europäisches Patentamt
European Patent Office
Office européen des brevets

(11) Publication number: **0 189 002 B1**

(12) **EUROPEAN PATENT SPECIFICATION**

(45) Date of publication of patent specification: **03.03.93**

(51) Int. Cl.5: **A61K 31/445**, A61K 31/46, C07D 451/14

(21) Application number: **85810595.0**

(22) Date of filing: **13.12.85**

(54) **Treatment of gastrointestinal disorders using 5-HT3 antagonists.**

(30) Priority: **20.12.84 DE 3446484**
**02.04.85 CH 1414/85**
**24.06.85 CH 2667/85**
**19.07.85 CH 3129/85**
**19.07.85 CH 3130/85**
**07.08.85 CH 3382/85**
**07.08.85 CH 3383/85**
**02.09.85 DE 3531281**
**02.09.85 DE 3531282**

(43) Date of publication of application:
**30.07.86 Bulletin 86/31**

(45) Publication of the grant of the patent:
**03.03.93 Bulletin 93/09**

(84) Designated Contracting States:
**AT BE CH DE FR GB IT LI LU NL SE**

(56) References cited:
**EP-A- 0 013 138**
**EP-A- 0 068 700**
**EP-A- 0 069 481**
**EP-A- 0 069 482**
**EP-A- 0 096 524**
**EP-A- 0 101 641**
**EP-A- 0 102 195**
**EP-A- 0 201 165**
**WO-A-40/3281**
**BE-A- 890 962**
**DE-A- 3 322 574**

(73) Proprietor: **SANDOZ AG**
**Lichtstrasse 35**
**CH-4002 Basel(CH)**
(84) Designated Contracting States:
**BE CH FR GB IT LI LU NL SE**

(73) Proprietor: **SANDOZ-PATENT-GMBH**
**Humboldtstrasse 3**
**W-7850 Lörrach(DE)**
(84) Designated Contracting States:
**DE**

(73) Proprietor: **SANDOZ-ERFINDUNGEN Verwaltungsgesellschaft m.b.H.**
**Brunner Strasse 59**
**A-1235 Wien(AT)**
(84) Designated Contracting States:
**AT**

(72) Inventor: **Buchheit, Karl-Heinz**
**Hermann-Albrecht-Strasse 10a**
**W-7850 Lörrach(DE)**

**EP 0 189 002 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ALLGEMEINE UND SPEZIELLE PHARMAKOLOGIE UND TOXIKOLGIE, 1980, Bibliografisches Inst., Mannheim, DE, Pages 149-152

J.Pharm.Pharmacol., vol. 37, no.9, September 1985, 664-667

Naunyn-Schmiedeberg's Arch. Pharmacol., vol. 329, no.1, March 1985, p. 36-41

Br. J. Pharmacol, vol. 87, suppl. 1986, p. 187p

Nature, vol. 316, n 6024, 11.07.1985, p. 126-131

Pharmacol., vol. 25, 1982, p. 61-72

Martindale The Extra Pharmacopoeia, 28th Ed., 1982, p. 964-966

Neuropharmacology, vol. 23, no.12B, December 1984, p. 1503-1510

## Description

The invention relates to medicaments suitable for the treatment of gastrointestinal disturbances containing bicyclic heterocyclic carboxylic acid esters and amides of cyclic alcohols or amines having nitrogen as a ring atom. These compounds are referred to hereinafter as compounds of the invention.

Belgian Patent Nos. 897,117;900,425 and 901,274 disclose some of such acids and amides which are stated to have serotonin M antagonistic activity (also known as 5-$HT_3$ antagonism).

The invention relates especially to the use of compounds of formula I

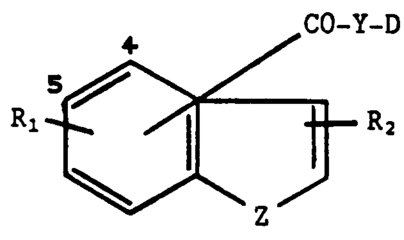

I

wherein the group -CO-Y-D is in one of the positions 3, 4 or 5 of the bicyclic ring

| | |
|---|---|
| Z | is -$NR_3$-, or -S-; |
| $R_1$ and $R_2$ | , independently, are hydrogen, halogen, $(C_{1-4})$alkyl or $(C_{1-4})$alkoxy, $R_1$ being in position 4 or 5. |
| $R_3$ | is hydrogen of $(C_{1-4})$alkyl |
| Y | is -O- or -NH-; and |
| D | is a group of formula VI |

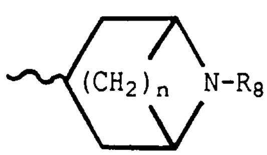

VI

wherein n is 2, 3 or 4; and

$R_8$ is hydrogen, $(C_{1-4})$alkyl, or benzyl; or a pharmaceutically acceptable acid addition or quaternary ammonium salt thereof in the manufacture of medicaments suitable for the treatment of serotonin induced gastrointestinal disturbances.

The essential antagonistic action against 5-HT of the preferred compound ICS 205-930, indol-3-yl carboxylic acid endo-8-methyl-8-aza-bicyclo-[3,2,1]oct-3-yl ester on the rabbit vagus, rabbit heart and guinea pig ileum has been described (P. Donatsch et al., Br. J. Pharmacol. 1984, 81, 348), and also its topical application to humans as the first 5-$HT_3$ antagonist. It was stated to be a competitive antagonist of the 5-$HT_3$ receptors in the guinea pig ileum on the basis of the effect of the compound in counteracting 5-HT induced spasms, these spasms being prolonged and permanent contractions. Moreover, no analysis was disclosed of its action in the guinea pig ileum.

Several drugs e.g. metoclopramide are known for the treatment of gastrointestinal disorders (gut motility disorders). Action has been ascribed to opiates (e.g. loperamide), dopaminergic compounds (e.g. metoclopramide) although for most drugs the mode of action is not well understood (see J. R. Malegeleda Scand. J. Gastroenterology, 19, Suppl. 96, 1984, pp. 115-121). Thus metoclopramide has been shown to have an $IC_{50}$ of about 170 nM/litre in binding studies with dopamine sites with tritiated haloperidol.

European Patent Publication 13138 discloses benzoic acid amides of piperidylamines having an alkylene bridge across ring positions 2 and 6. The compounds are stated to be active inter alia in increasing the intragastric pressure in the rat, in reversing the apomorphine induced delay in gastric emptying in the rat, and inhibiting the apormorphine-induced emetic effect in the rat. The compounds are indicated to be dopamine antagonists and useful in the treatment of impaired gastrointestinal motility, such as retarded gastric emptying, dyspepsia, flatulence, oesophageal reflux, peptic ulcer and emesis.

The compounds of the present invention in general do not significantly affect apomorphine-induced symptoms. They show on the other hand a long lasting and potent effect in the treatment of serotonin-induced gastrointestinal disturbances by action on 5-$HT_3$ receptors. The compounds of the invention have

thus a different mechanism of action to that stated for those in European Patent Publication 13138 and are well tolerated.

Peristaltic movements (Peristaltis) are coordinated contractions and are necessary for the proper functioning of the gastrointestinal system. We have now found that activation of 5-$HT_3$ receptors is involved in provocation of abnormal peristaltic movements and associated gastrointestinal disorders of motility and secretion. Also we have found that serotonin antagonists are useful in decreasing increased peristaltic movements in the intestines and increasing decreased peristaltic movements in the stomach, and are hence useful in the treatment of disorders of gastric motility.

We have further found that reflexes responsible for different kinds of intestinal reactions such as smooth muscle contractions, secretion by mucosal cells and/or dilation of intestinal blood vessels, a pre-requisite for the secreation of fluids, are stimulated by stimulation of 5-$HT_3$ receptors located on afferent sensory neurons by 5-$HT_3$ released on the serosal side and are blocked by 5-$HT_3$ antagonists.

Preferred compounds of the invention include:-

Indol-3-yl carboxylic acid endo-8-methyl-8-aza-bicyclo[3,2,1]-oct-3-yl ester [hereinafter compound E]

Benzo[b]thiophen-3-yl carboxylic acid endo-9-methyl-asa-bicyclo-[3,3,1]non-3-yl ester [hereinafter compound F]

5-fluoro-1-methyl-indol-3-yl carboxylic acid endo-9-methyl-9-aza-bicyclo[3,3,1]non-3-yl ester [hereinafter compound G and also the compound No. 36].

The action of the compounds of the invention is shown in the following tests:-

## Test A

The compounds of the invention facilitate field stimulation-induced contractions in muscle strips from different parts of the guinea pig stomach and are therefore indicated to increase decreased peristaltic movements in the stomach and to enhance gastric emptying in vivo. The test is effected as follows:-

Male Dunkin-Hartley guinea pigs, 340-450 g, which had been starved overnight, were killed by cervical transsection and the stomach removed and placed in Krebs-Henseleit solution (NaCl 118.0, KCl 4.75, $KH_2PO_4$ 1.2, $MgSO_4$ 1.2, $CaCl_2$ 2.5, glucose 10 mM). Segments were taken from the body (approximately 20 mm long, 3-4 mm wide) with dissection in a plane suitable to investigate tension changes in the circular muscle layer. Tissues were placed in 30 ml organ baths containing oxygenated (95% $O_2$, 5% $CO_2$) Krebs-Henseleit solution at 37° C. One gram tension was applied to the tissues which were allowed to equilibrate for 45-60 min. before electrical stimulation. Intramural stimulation was achieved by using platinum wire electrodes placed approximately 5 mm apart, current being obtained from a Farnel Physiological stimulator. Tension changes were detected by Grass tension tranducers and displayed on a multichannel Grass recorder. A frequency-response curve was initially constructed in the absence of test substance and then in the presence of the test substance which was allowed a 45 min. pretreatment time. The second curve was related to the first to assess the degree of potentiation or antagonism. Tissues were stimulated for 30s at 5 min. intervals. Fresh tissues were used to assess such antagonist interaction. Appropriate solvent control experiments were carried out throughout the studies. Responses were measured as changes in gram tension, but to allow easier comparison between treatments, data were converted to show changes as percentage values. The significance of differences between control responses and those obtained in the presence of interacting drugs was assessed using the Mann-Whitney U test.

The compounds of the invention are active at about $10^{-7}$ M to about $10^{-9}$ Molar and induce frequency related increases in contraction responses.

Compound E produced a maximal response at $10^{-8}$ M and was 100 times more active than metoclopramide.

## Test B

The compounds of the invention also induce gastric emptying as indicated in standard in vivo tests, e.g. in conscious guinea pigs having stomachs made atonic (i.e. having decreased peristaltic movements) by fasting and wherein the passage of glass spheroids was observed by X-ray techniques. The experiment is effected as follows:

Food was withdrawn for 14 hours before the measurement of gastric emptying. The experiment was conducted under low illumination which minimal noise and disturbance, and was carried out only by those experimentors who had daily contact with the guinea pigs and who carried out the initial training to accustom the guinea pigs to handling. Therefore, animals were subject to minimal stress. Measurement of gastric emptying was achieved by X-ray location (50 KV, 30 mA, 0.5-0.9 s) using Kodak plates (NS-2T, 13 x

18 cm) of polystyrene-coated barium sulphate spheroids (approximately 30, 1 mm in diameter) which were swallowed by the guinea pigs when placed in the back of the mouth in 0.2 ml of 1% carboxymethylcellulose with 0.05 ml glycerin to initiate prompt and voluntary swallowing. The passage of the speroids was followed for 3-4 h: during this period animals were placed in their normal housing cages and were only removed 5 min prior to X-ray (at 30 - 60 min. intervals) when they were placed in an individual perspex holding cage which held the animal comfortably in a stable position. The holding cage was correctly shaped (33 x 15 cm, and 13 cm high) to hold a 450-550 g guinea pig between foam - lined sides and an animal trained to entering the cage would do so and remain quiet and unstressed during the X-ray procedure. Gastric emptying was measured as the number of spheroids leaving the stomach.

Six guinea pigs were used at each dose level of drug and responses compared to those of guinea pigs receiving the appropriate vehicle. The significance of differences between drug and control responses was assessed using the Mann Whitney U test.

The compounds of the invention are active at doses of about 0.01 to about 1 mg/kg i.p. in enhancing gastric emptying. Compound E was about 50 times more active than metoclopramide.

The effect of the compounds increasing gastric emptying indicates an increased tonus on gastrointestinal tract.

The compounds of the invention are also useful in inhibiting increased peristaltic movements in the intestines as indicated in the following tests.

**Test C**

In a further test the inhibition of the increase in gastrointestinal motility induced by 5-hydroxytryptophan (5-HTP) by the compounds of the invention was observed.

Male NMRI mice (18-32 g in weight) are deprived of food 20 hours before the test. Water is not limited. The animals are separated by a barrier from their straw and access to feces. At the beginning of the test the animals are separated in individual single cages and water is removed.

All animals were treated with the test compound or saline. I.p. administration was used. Injection volume was 0.1 ml/10 g. 30 minutes after pre-treatment 5-HTP or saline was administered. Injection volume 0.1 ml /10 g. Dose 3 mg/kg. At the same time charcoal was administered perorally (10% suspension in water; 0.1 ml/10g). 45 minutes after the start of the experiment the animals were killed. The intestines from the stomach to the rectum were examined. For each animal the transit distance of the front of the charcoal meal along the intestine was measured. The distance was ascertained as a percentage of the whole intestinal length. Groups of at least 3 animals were used.

The compounds of the invention inhibit 5-HTP induced motility at doses of about 10 to 100 µg/kg i.p.

| | $ED_{50}$ i.p. |
|---|---|
| Compound E | 70 µg/kg |
| Compound F | 25 µg/kg |
| Compound G | 2 µg/kg |

**Test D**

In a further test the compounds are shown to inhibit cholera toxin-induced secretory diarrhea which leads to increased perstaltic motility.

**Method**

Male NMRI mice (20-30 g) were deprived of food for 24 hours, but had free access to water. For the duration of the experiment water was subsequently withdrawn. Saline or the test compound were administered intraperitoneally. Four dose levels of each drug were investigated and each dose was given to 5 animals. One hour after pre-treatment with the drug the animals were challenged with 200 µg of pure cholera toxin p.o. through a tube to each stomach followed by 2 ml of the above Tyrode solution. Three hours later the administration of the test compounds was repeated. Four hours after the cholera toxin challenge the animals were killed and the content of the whole intestine determined by weighing.

| Adminstration protocol | |
|---|---|
| 0 hours | Administration of test compound |
| 1 hour | Administration of cholera toxin |
| 3 hours | Repeated addition of test compound |
| 4 hours | sacrifice of animals. |

The intestinal contents are usually increased under the influence of cholera toxin. This effect was reduced by 50% by the test compounds, in particular by 50% at a dose of 100 to 500 micrograms/kg. An increase in the dose of compound E did not lead to a reduction in the stomach contents.

The isolated longitudinal muscle of the guinea pig ileum with its adhering myenteric plexus is a well established model which permits investigation of the mechanism of action of various neurtransmitters. It does not, however, indicate by itself action of the compounds on peristaltic movements.

**Method**

Male guinea pigs (200-400 g) were killed by a blow on the head and exsanguinated. A length of small intestine was removed about 2 cm from the ileo-caecal valve. The mesentery was carefully removed and the ileum was stretched over a glass rod. By stroking tangentially away from the mesenteric attachment with a wad of cotton wool, the longitudinal muscle layer was separated and stripped from the unterlying circular muscle. Longitudinal muscle strips, 3-4 cm length, were mounted in a 10 ml organ bath containing Tyrode solution at 37° C and bubbled with 5% carbon dioxide in oxygen. The Tyrode solution was of the following composition (mmol/1):NaCl, 137.0; $CaCl_2$, 1.8; KCl, 2.7; $MgCl_2$, 1.05; $NaHCO_3$, 11.9; $NaH_2PO_4$, 0.4; glucose, 5.6. The strips were placed under a resting tension of 500 mg. Contractions were recorded with an isotonic pendulum lever. After equilibration for 30 min a set concentration of carbachol was applied in 10 ml intervals until a consistant reaction was achieved.

**Production of the concentration/reaction curve**

Non-cumulative concentration-response curves for 5-HT were established by adding increasing concentrations of the agonist to the organ bath at intervals of at least 15 min. Preceding experiments showed that the intervals were long enough to avoid tachyphylaxis. Each concentration was left in contact with the tissue for 1 min. Each strip was only used to record two concentration-response curves; the first for 5-HT alone and the second for 5-HT in the presence of a set concentration of antagonist, each strip thus serving as its own control. Antagonists were allowed to preequilibrate for at least 10 min prior to addition of 5-HT. The contractions expressed as percentage of the maximal response to 5-HT obtained from several preparations were plotted as mean values in order to obtain log-concentration-response curves. Inhibition constants were expressed in the form of $pA_2$ values which were graphically determined according to conventional methods (Arunlakshana and Schild, 1959. Mc Kay 1978).

In this test 5-HT elicits a concentration - dependent contractile effect. 5-HT induces its major contractile effects in the longitudinal muscle strip of the guinea pig ileum by releasing substance P from nerve endings within this tissue. Its effect is mediated by two different 5-HT receptors. At low concentrations 5-HT activates a neuronal receptor which causes substance P release. The liberated substance P activates neuronal substance P receptors and this causes the release of acetylcholine which subsequently activates muscarinic receptors located on smooth muscle cells and brings about contraction. At higher concentrations 5-HT activates a second neuronal receptor which results in release of sufficient quantities of substance P to cause activation of substance P receptors on smooth muscle cells and thereby cause contraction.

The compounds of the invention block preferentially the low affinity 5-HT receptors thereby inhibiting 5-HT-induced contraction e.g. at concentrations from about $10^{-7}$ to about $10^{-9}$ mol/litre.

| | high affinity receptor | low affinity receptor |
|---|---|---|
| Compound E | $pD'_2 = 5.7$ | $pA_2 = 7.9$ |
| Compound F | $pD'_2 = 5.9$ | $pA_2 = 9.4$ |

In this test the ratio between the $pA_2$ value and the $pD'_2$ value is conveniently greater than 100, more preferably 1000.

The compounds have little affinity for the high affinity 5-HT receptor indicated to be responsible for controlling secretion and motility processes involving acetylcholine release, but have greater affinity for the low affinity serotonin receptor sub-type which is indicated to be involved in pathophysiological abnormal peristaltic movements conditions such as gastroparesis (lack of stomach motility) or diarrhea.

The compounds of the invention preferentially inhibit the 5-HT reflex without affecting the basal reflex activity and an increase in peristaltic movements. This is in contrast to drugs like atropine which paralyse the whole reflex, and metoclopramide which has dopamine agonist action.

In the charcoal meal test in the rat at doses of over 50 mg/kg s.c. of the compounds of the invention the influence on normal gastric motility is not significant.

The compounds of the invention have insignificant action on dopamine binding sites, e.g. having an $IC_{50}$ of 100 mM/litre or more using tritiated haloperidol.

Gastrointestinal disorders which result from increased peristaltic movements in the intestinal tract or from decreased peristaltic movements in the stomach are therefore indicated to be treated by blockade of $5\text{-}HT_3$ neuronal receptors. The compounds of the invention are indicated for use in the treatment of gastrointestinal disorders. The compounds also inhibit or prevent the action of 5-HT on low affinity 5-HT receptors in the gastrointestinal tract, and therefore are indicated for use in the treatment of gastroparesis or disturbances of motility. They are also indicated for use in gastrointestinal disorders where an abnormal increase in the synthesis or liberation of 5-HT from the enterochromaffin cells or neurons has taken place without significantly affecting basal secretion or motility. They are also indicated to be well tolerated.

The compounds of the invention are therefore useful in the treatment of gastrointestinal disturbances which require antagonism to $5\text{-}HT_3$ receptors.

The medicaments prepared according to the invention containing compounds of formula I are suitable for the treatment of gastro-intestinal disturbances selected from gastritis, peptic ulcer, biliary dyskinesia, spastic colon, appendicitis, irritable bowel syndrome, Crohn's disease, ulcerative colitis, carcinoid syndrome and diarrhea of different genesis, e.g. secretory diarrhea, bacteria-induced diarrhea, choleric diarrhea, traveller's diarrhea, psychogenic diarrhea or oesophageal motility disturbances, achalasis, hiatus hernia, cardia insufficiency, gastrooesophageal and gastroduodenal reflux, stomach hypotonia, pylorus hyperplasia, paralytic ileus and Hirschsprung's disease.

For the above indications, the exact dosage of compounds of formula I will, of course, vary depending on the compound employed, mode of administration and treatment disired. In general, satisfactory results are obtained in doses from 0.01 to 10 mg/kg of compounds of formula I. For the larger primates, in particular humans, an indicated daily dosage is in the range from 0.5 mg to 500 mg (e.g. 20 to 200, or 20 to 100 mg or 20 to 400 mg), of a compound of formula I conveniently administered, for example, in divided doses 2 to 4 times a day. Unit dosage forms contain, for example, from 0.1 mg to 250 mg of the compound. If desired, the medicaments may be administered in a single dose for acute therapy.

From compounds of formula I compounds E and F are the preferred compounds.

The compounds of formula I in form of medicaments may be administered in similar manner to known standards for use in these indications. The suitable daily dosage for a particular compound will depend on a number of factors, such as its relative potency of activity.

On the basis of the activity of the compound E in the above tests, an indicated daily dose for the compound E is from 5 to 20 mg p.o. for larger primates such as humans.

The compounds of formula I may be present in the medicaments in free base form or in pharmaceutically acceptable acid addition salt form or in a quaternary ammonium salt form. Such salts may be prepared in conventional manner and are in general known. They exhibit the same order of activity as the free base form and medicaments comprise a compound of formula I in free base or pharmaceutically acceptable acid addition salt form or quaternary ammonium salt form in association with pharmaceutical carrier or diluent. Such medicaments may be manufactured in conventional manner.

The specific compounds maintained hereinafter are preferred to be present in the medicaments in the salt form mentioned in the above Belgian patents, e.g. Compound E as the hydrochloride. The compounds F and G have previously only been disclosed in the free base form. We have now found that compounds F and G are preferably used e.g. in medicaments in the form of the hydrochloride of compound F (m.pt. 242-243 °C) and hydrogen maleate of compound G (m.pt. 171-172 °C). These forms have been found to have especially advantageous properties, e.g. from the solubility and stability points of view.

The medicaments may be administered by any conventional route, in particular enterally, preferably orally, e.g. in the form of tablets or capsules or parenterally, e.g. in the form of injectable solutions or suspensions.

Suitable pharmaceutical carriers and diluents for oral administration include polyethylene glycol, polyvinylpyrrolidone, mannitol, lactose etc. granulating agents, and disintegrating agents such as starch and

algenic acid, binding agents such as stearic and gelatine, lubricating agents such as magnesium stearate. Stearic acid and talc. Suspensions may contain conserving agents like ethyl p-hydroxy-benzoate, suspending agents such as methyl-cellulose, tensides etc. For parenteral forms the compositions are preferably buffered, aqueous solutions (pH between 4 and 5).

The following examples illustrate the invention.

**Example 1 : Tablets for oral administration**

Tablets containing the constituents as specified below were produced in conventional manner and are used in the indication specified above.

| | |
|---|---|
| Compound E in form of hydrochloride (corresponding to 15 mg free base) | 16.9 mg |
| Hydroxy-propyl-cellulose | 1.2 mg |
| Corn Starch | 12.0 mg |
| Lactose | 92.8 mg |
| Silica | 0.6 mg |
| Magnesium stearate | 1.5 mg |
| Tablet weight | 125.0 mg |

**Example 2 : Capsules for oral administration**

Capsules containing the constituents as specified below are produced in conventional manner and are used in the indications specified above.

| | |
|---|---|
| 1-methyl-indol-3-yl-carboxylic acid-N(endo-9-methyl-9-aza-bicyclo[3,3,1]non-3-yl) amid in the form of the hydrochloride (corresponding to 15 mg base) | 16.8 mg |
| Lactose | 28.7 mg |
| Silica | 1.5 mg |
| Magnesium stearate | 3.0 mg |
| Capsule Content weight of | 300.0 mg |

**Example 3 : Injection solution for i.v. administration**

A composition for injection is made up in conventional manner and is used at a dose of 10 mg a day.

| | **A** | **B** | **C** |
|---|---|---|---|
| Compound E in form of hydrochloride | 1.13[1)] | 2.256[2)] | 11.282[3)] |
| Acetic acid (99 to 100%)* | 1.2 | 0.6 | 0.6 |
| Sodium acetate 3.$H_2O$* | 1.8 | 3.18 | 3.18 |
| Sodium chloride | 8 | 7.5 | 6.5 |
| Water for injection to | 1.0 ml | | |

1) = 1 mg free base
2) = 2 mg free base
3) = 10 mg free base
pH value 4.3; Buffer used* 1/30 molar

**Example 4 : Capsules for oral administration**

5 mg and 15 mg capsules (A and B respectively) containing the constituents as specified below were produced in conventional manner and are used in the indication specified above 2-4 times a day in the case

of A and once a day in the case of B.

| | A mg | B mg |
|---|---|---|
| Compound E in form of hydrochloride | 5.641 | 16.92 |
| Lactose 200 mesh | 84.929 | 79.29 |
| Lactose 100 mesh | 84.43 | 79.29 |
| Corn starch | 120.00 | 120.00 |
| Silica | 1.5 | 1.5 |
| Magnesium stearate | 3.0 | 3.0 |
| | 300 mg | 300 mg |

Capsules containing other weights can be formulated in conventional manner.

The active agents in Examples 1 to 3 may be replaced by the following compounds of formula I wherein:

| Compound No. | $R_1$ | $R_2$ | Z | -CO-Y-D group in position | Y | Conf. | D = n (VI) | $R_8$ |
|---|---|---|---|---|---|---|---|---|
| 1 | H | H | NH | 3 | NH | endo | 3 (VI) | Me |
| 2 | 5-F | H | NMe | 3 | NH | endo | 3 (VI) | Me |
| 3 | H | 2-Cl | NH | 3 | 0 | endo | 2 (VI) | Me |
| 4 | H | 2-OMe | NH | 3 | 0 | endo | 2 (VI) | Me |
| 5 | H | 3-J | NH | 4 | 0 | endo | 2 (VI) | Me |
| 6 | H | H | NH | 4 | 0 | endo | 2 (VI) | Me |
| 7 | H | H | NH | 4 | 0 | endo | 3 (VI) | Me |
| 8 | 5-Cl | H | NH | 3 | 0 | endo | 2 (VI) | Me |
| 9 | 4-OMe | H | NH | 3 | 0 | endo | 2 (VI) | Me |
| 10 | 5-OMe | H | NH | 3 | 0 | endo | 2 (VI) | Me |
| 11 | H | H | NMe | 3 | 0 | endo | 2 (VI) | Me |
| 12 | H | H | NH | 3 | 0 | exo | 2 (VI) | Me |
| 13 | 5-F | H | NH | 3 | NH | endo | 2 (VI) | Me |
| 14 | H | H | NMe | 3 | NH | endo | 2 (VI) | Me |
| 15 | H | 2-Me | NH | 3 | NH | endo | 2 (VI) | Me |

| Compound No. | $R_1$ | $R_2$ | Z | -CO-Y-D group in position | Y | Conf. | D = n (VI) | $R_8$ |
|---|---|---|---|---|---|---|---|---|
| 16 | H | H | NH | 3 | NH | exo | 2 (VI) | Me |
| 17 | H | H | NH | 3 | NH | endo | 2 (VI) | Me |
| 18 | 5-Cl | H | NH | 3 | H | endo | 2 (VI) | Me |
| 19 | H | H | NH | 3 | 0 | endo | 3 (VI) | Me |
| 20 | H | H | NMe | 3 | 0 | endo | 3 (VI) | Bz |
| 21 | 5-F | H | NH | 3 | 0 | endo | 3 (VI) | Bz |
| 22 | H | H | S | 3 | 0 | endo | 3 (VI) | Me |
| 23 | H | H | S | 3 | NH | endo | 3 (VI) | Me |
| 24 | H | H | NH | 3 | NH | exo | 4 (VI) | Me |
| 25 | H | H | NH | 3 | 0 | exo | 4 (VI) | Me |
| 26 | H | H | NH | 3 | 0 | endo | 3 (VI) | Me |
| 27 | H | H | NH | 3 | 0 | endo | 2 (VI) | n-Pr |
| 28 | H | H | NH | 3 | 0 | exo | 2 (VI) | Bz |
| 29 | H | H | NH | 3 | 0 | endo | 2 (VI) | Bz |

| Compound No. | $R_1$ | $R_2$ | Z | -CO-Y-D group in position | Y | Conf. | D = n (VI) | $R_8$ |
|---|---|---|---|---|---|---|---|---|
| 30 | H | H | NH | 3 | 0 | endo | 2 (VI) | H |
| 31 | 5-F | H | NH | 3 | 0 | endo | 3 (VI) | H |
| 32 | H | H | NMe | 3 | 0 | endo | 3 (VI) | H |
| 33 | H | H | NH | 3 | 0 | endo | 3 (VI) | H |
| 34 | 5-Me | H | NH | 3 | 0 | endo | 3 (VI) | Me |
| 35 | H | 2-Me | NH | 3 | 0 | endo | 3 (VI) | Me |
| 36 | 5-F | H | NMe | 3 | 0 | endo | 3 (VI) | Me |
| 37 | 5-F | H | NH | 3 | 0 | endo | 3 (VI) | Me |
| 38 | 5-F | H | NMe | 3 | 0 | endo | 3 (VI) | Bz |
| 39 | H | H | NMe | 3 | 0 | endo | 3 (VI) | Me |
| 40 | 5-Me | H | NH | 3 | NH | endo | 3 (VI) | Me |
| 41 | H | H | NH | 5 | 0 | endo | 2 (VI) | Me |
| 42 | H | H | NH | 5 | 0 | endo | 3 (VI) | Me |
| 43 | H | 3-I | NH | 5 | 0 | endo | 3 (VI) | Me |
| 44 | H | H | NH | 4 | NH | exo | 2 (VI) | Me |
| 45 | H | H | NH | 4 | NH | endo | 2 (VI) | Me |
| 46 | H | H | NH | 5 | H | endo | 2 (VI) | Me |

## Claims

**1.** Use of a compound of formula

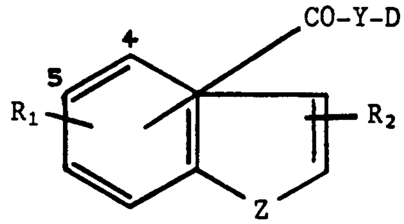

I

wherein the group -CO-Y-D is in one of the positions 3, 4 or 5, of the bicyclic ring

| | |
|---|---|
| Z | is $-NR_3-$, or -S-; |
| $R_1$ and $R_2$ | , independently, are hydrogen, halogen, $(C_{1-4})$alkyl or $(C_{1-4})$alkoxy, $R_1$ being in position 4 or 5 |
| $R_3$ | is hydrogen or $(C_{1-4})$alkyl |
| Y | is -O- or -NH-; and |
| D | is a group of formula VI |

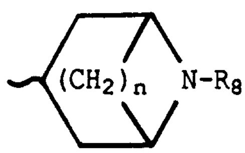

VI

wherein n is 2, 3 or 4; and

$R_8$ is hydrogen, $(C_{1-4})$alkyl, or benzyl; or a pharmaceutically acceptable acid addition or quaternary ammonium salt thereof in the manufacture of medicaments suitable for the treatment of serotonin induced gastrointestinal disturbances.

**2.** Use according to claim 1 wherein the compound used is indol-3-yl carboxylic acid endo-8-methyl-8-aza-bicyclo[3,2,1]oct-3-yl ester.

**3.** Use of compounds according to claim 1 in the manufacture of a medicament suitable for the treatment of gastro-intestinal disturbances selected from gastritis, peptic ulcer, biliary dyskinesia, spastic colon, appendicitis, irritable bowel syndrome, Crohn's disease, ulcerative colitis, carcinoid syndrome and diarrhea of different genesis, e.g. secretory diarrhea, bacteria-induced diarrhea, choleric diarrhea, traveller's diarrhea, psychogenic diarrhea or oesophageal motility disturbances, achalasis, hiatus hernia, cardia insufficiency, gastrooesophageal and gastroduodenal reflux, stomach hypotonia, pylorus hyperplasia, paralytic ileus and Hirschsprung's disease.

**4.** Use according to claim 3 wherein the compound used is indol-3-yl carboxylic acid endo-8-methyl-8-aza-bi-cyclo[3,2,1]oct-3-yl ester.

**5.** Use according to claim 3 wherein the gastro-intestinal disturbance is gastro-oesophageal reflux disorder.

**6.** Use according to claim 5 wherein the compound used is indol-3-yl carboxylic acid endo-8-methyl-8-aza-bicyclo[3,2,1]oct-3-yl ester.

**7.** Use according to claim 3 wherein the gastro-intestinal disturbance is spastic colon.

**8.** Use according to claim 7 wherein the compound used is indol-3-yl carboxylic acid endo-8-methyl-8-aza-bicyclo[3,2,1]oct-3-yl ester.

**9.** Use according to claim 7 wherein the gastrointestinal disturbance is irritable bowel syndrome.

**10.** Use according to claim 9 wherein the compound used is indol-3-yl carboxylic acid endo-8-methyl-8-aza-bi-cyclo[3,2,1]oct-3-yl ester.

**11.** Use according to claim 3 wherein the gastrointestinal disturbance is diarrhea.

**12.** Use according to claim 11 wherein the compound used is indol-3-yl carboxylic acid endo-8-methyl-8-aza-bi-cyclo[3,2,1]oct-3-yl ester.

**13.** Use according to claim 11 wherein the gastrointestinal disturbance is secretory diarrhea.

**14.** Use according to claim 13 wherein the compound used is indol-3-yl carboxylic acid endo-8-methyl-8-aza-bi-cyclo[3,2,1]oct-3-yl ester.

**15.** Use according to claim 11 wherein the gastrointestinal disturbance is traveller's diarrhea.

**16.** Use according to claim 15 wherein the compound used is indol-3-yl carboxylic acid endo-8-methyl-8-aza-bicyclo[3,2,1]oct-3-yl ester.

**17.** Use according to claim 11 wherein the gastrointestinal disturbance is bacterial induced diarrhea.

**18.** Use according to claim 17 wherein the compound used is indol-3-yl carboxylic acid endo-8-methyl-8-aza-bicyclo[3,2,1]oct-3-yl ester.

**Patentansprüche**

**1.** Verwendung einer Verbindung der Formel I

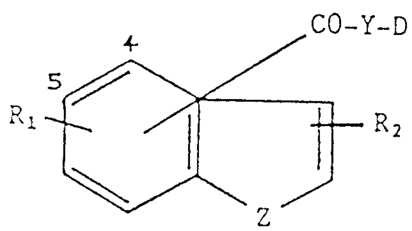

I

worin sich der Rest CO-Y-D in einer der Stellungen 3,4 oder 5 des bicyclischen Ringes befindet

| | |
|---|---|
| Z | für $-NR_3-$,oder -S- steht |
| $R_1$ und $R_2$ | unabhängig voneinander Wasserstoff,Halogen,$(C_{1-4})$Alkyl oder $(C_{1-4})$Alkoxy bedeuten wobei sich $R_1$ in den Stellungen 4 oder 5 befindet |
| $R_3$ | Wasserstoff oder $(C_{1-4})$Alkyl bedeutet |
| Y | für -O- oder -NH- steht |
| D | eine Gruppe der Formel VI |

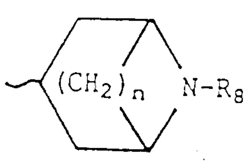

VI

bedeutet, worin n 2,3 oder 4 ist und

$R_8$ für Wasserstoff,$(C_{1-4})$Alkyl oder Benzyl steht oder eines pharmazeutisch annehmbaren Säureadditionssalzes oder eines quaternären Ammoniumsalzes dieser Verbindung zur Herstellung eines Medikamentes das zur Behandlung von serotonininduzierten gastrointestinalen Störungen geeignet ist.

2. Verwendung gemäss Anspruch 1 worin die verwendete Verbindung der Indol-3-yl carbonsäure endo -8-methyl-8-aza-bicyclo[3,2,1]oct-3-yl ester ist

3. Verwendung von Verbindungen gemäss Anspruch 1 zur Herstellung von Medikamenten die geeignet sind zur Behandlung von gastrointestinalen Störungen die ausgewählt sind aus Gastritis, Magengeschwüren, Gallendyskinesien,spastischem Colon, Appendicitis, Reizdarm, Morbus Crohn,Colitis Ulcerosa, Carcinoidsyndrom und Diarrhöen verschiedenen Ursprungs wie sekretorische Diarrhöe, durch Bakterien verursachte Diarrhöe, durch Cholera verursachte Diarrhöe, Reisediarrhöe, psychogene Diarrhöe oder Bewegungsstörungen der Speiseröhre, Achalasie, Hiatushernie, Cardiainsuffizienz, gastrooesophagaler und gastroduodenaler Reflux, Magenhypotonie, Pylorushyperplasie, paralytischer Ileus und Morbus Hirschsprung.

4. Verwendung gemäss Anspruch 3 worin die verwendete Verbindung der Indol-3-yl carbonsäure endo-8-methyl-8-aza-bicyclo[3,2,1]oct-3-yl ester ist.

5. Verwendung gemäss Anspruch 3 worin die gastrointestinale Störung eine Störung des gastrooesophagalen Refluxes ist.

6. Verwendung gemäss Anspruch 5 worin die verwendete Verbindung der Indol-3-yl carbonsäure endo-8-methyl-8-aza-bicyclo[3,2,1]oct-3-yl ester ist.

7. Verwendung gemäss Anspruch 3 worin die gastrointestinale Störung ein spastischer Colon ist.

8. Verwendung gemäss Anspruch 7 worin die verwendete Verbindung der Indol-3-yl carbonsäure endo-8-methyl-8-aza-bicyclo[3,2,1]oct-3-yl ester ist.

9. Verwendung gemäss Anspruch 7 worin die gastrointestinale Störung ein Reizdarm ist.

10. Verwendung gemäss Anspruch 9 worin die verwendete Verbindung der Indol-3-yl carbonsäure endo-8-methyl-8-aza-bicyclo[3,2,1]oct-3-yl ester ist.

11. Verwendung gemäss Anspruch 3 worin die gastrointestinale Störung eine Diarrhöe ist.

12. Verwendung gemäss Anspruch 11 worin die verwendete Verbindung der Indol-3-yl carbonsäure endo -8-methyl-8-aza-bicyclo[3,2,1]oct-3-yl ester ist.

13. Verwendung gemäss Anspruch 11 worin die gastrointestinale Störung eine sekretorische Diarrhöe ist.

14. Verwendung gemäss Anspruch 13 worin die verwendete Verbindung der Indol-3-yl carbonsäure endo -8-methyl-8-aza-bicyclo[3,2,1]oct-3-yl ester ist.

15. Verwendung gemäss Anspruch 11 worin die gastrointestinale Störung eine Reisediarrhöe ist.

16. Verwendung gemäss Anspruch 15 worin die verwendete Verbindung der Indol-3-yl carbonsäure endo-8-methyl-8-aza-bicyclo[3,2,1]oct-3-yl ester ist.

17. Verwendung gemäss Anspruch 11 worin die gastrointestinale Störung eine bakterieninduzierte Diarrhöe ist.

18. Verwendung gemäss Anspruch 17 worin die verwendete Verbindung der Indol-3-yl carbonsäure endo-8-methyl-8-aza-bicyclo[3,2,1]oct-3-yl ester ist.

## Revendications

1. L'utilisation d'un composé de formule

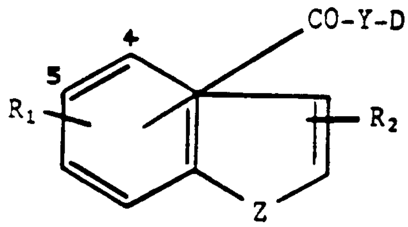

I

dans laquelle le groupe -CO-Y-D est situé à l'une des positions 3, 4 ou 5 du système bicyclique,

Z signifie $-NR_3-$, ou -S-,

$R_1$ et $R_2$ , indépendamment, signifient l'hydrogène, un halogène ou un groupe alkyle en $C_1$-$C_4$ ou alcoxy en $C_1$-$C_4$ , $R_1$ étant en position 4 ou 5,

$R_3$ signifie l'hydrogène ou un groupe alkyle en $C_1$-$C_4$,

Y signifie -O- ou -NH-, et

D signifie un groupe de formule VI

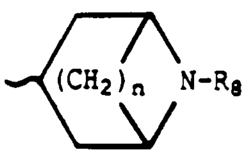

VI

dans laquelle n signifie 2, 3 ou 4, et

$R_8$ signifie l'hydrogène ou un groupe alkyle en $C_1$-$C_4$ ou benzyle,

ou un sel d'addition d'acide ou un sel d'ammonium quaternaire pharmaceutiquement acceptables de ce composé,

pour la préparation de médicaments appropriés pour le traitement des troubles gastro-intestinaux provoqués par la sérotonine.

2. L'utilisation selon la revendication 1, caractérisée en ce que le composé utilisé est l'ester endo-8-méthyl-8-aza-bicyclo[3,2,1]oct-3-ylique de l'acide indole-3-yl-carboxylique.

3. L'utilisation des composés selon la revendication 1 pour la préparation d'un médicament approprié pour le traitement des troubles gastro-intestinaux choisis parmi la gastrite, l'ulcère gastro-duodénal, la dyskinésie biliaire, le côlon irritable, l'appendicite, le syndrome des intestins irritables, la maladie de Crohn, la colite ulcéreuse, le syndrome carcinoïde et les diarrhées d'origines diverses, par exemple la diarrhée secrétoire, la diarrhée provoquée par des bactéries, la diarrhée du choléra, la diarrhée du voyageur, la diarrhée psychogène ou les troubles de la motilité oesophagienne, l'achalasie, la hernie hiatale, l'insuffisance du cardia , le reflux gastro-oesophagien et gastro-duodénal, l'hypotonie stomacale, l'hypertrophie du pylore, l'iléus paralytique et la maladie de Hirschsprung.

4. L'utilisation selon la revendication 3, caractérisée en ce que le composé utilisé est l'ester endo-8-méthyl-8-aza-bicyclo[3,2,1]oct-3-ylique de l'acide indole-3-yl-carboxylique.

5. L'utilisation selon la revendication 3, caractérisée en ce que le trouble gastro-intestinal est le trouble du reflux gastro-oesophagien.

6. L'utilisation selon la revendication 5, caractérisée en ce que le composé utilisé est l'ester endo-8-méthyl-8-aza-bicyclo[3,2,1]oct-3-ylique de l'acide indole-3-yl-carboxylique.

7. L'utilisation selon la revendication 3, caractérisée en ce que le trouble gastro-intestinal est le côlon irritable.

**8.** L'utilisation selon la revendication 7, caractérisée en ce que le composé utilisé est l'ester endo-8-méthyl-8-aza-bicyclo[3,2,1]oct-3-ylique de l'acide indole-3-yl-carboxylique.

**9.** L'utilisation selon la revendication 7, caractérisée en ce que le trouble gastro-intestinal est le syndrome des intestins irritables.

**10.** L'utilisation selon la revendication 9, caractérisée en ce que le composé utilisé est l'ester endo-8-méthyl-8-aza-bicyclo[3,2,1]oct-3-ylique de l'acide indole-3-yl-carboxylique.

**11.** L'utilisation selon la revendication 3, caractérisé en ce que le trouble gastro-intestinal est la diarrhée.

**12.** L'utilisation selon la revendication 11, caractérisée en ce que le composé utilisé est l'ester endo-8-méthyl-8-aza-bicyclo[3,2,1]oct-3-ylique de l'acide indole-3-yl-carboxylique.

**13.** L'utilisation selon la revendication 11, caractérisée en ce que le trouble gastro-intestinal est la diarrhée secrétoire.

**14.** L'utilisation selon la revendication 13, caractérisée en ce que le composé utilisé est l'ester endo-8-méthyl-8-aza-bicyclo[3,2,1]oct-3-ylique de l'acide indole-3-yl-carboxylique.

**15.** L'utilisation selon la revendication 11, caractérisée en ce que le trouble gastro-intestinal est la diarrhée du voyageur.

**16.** L'utilisation selon la revendication 15, caractérisée en ce que le composé utilisé est l'ester endo-8-méthyl-8-aza-bicyclo[3,2,1]oct-3-ylique de l'acide indole-3-yl-carboxylique.

**17.** L'utilisation selon la revendication 11, caractérisée en ce que le trouble gastro-intestinal est la diarrhée provoquée par des bactéries.

**18.** L'utilisation selon la revendication 17, caractérisée en ce que le composé utilisé est l'ester endo-8-méthyl-8-aza-bicyclo[3,2,1]oct-3-ylique de l'acide indole-3-yl-carboxylique.